# EUROPEAN PATENT SPECIFICATION

(11) **EP 1 465 672 B1**
(45) Date of publication and mention of the grant of the patent: **22.08.2007**
(21) Application number: 03700870.3
(22) Date of filing: 16.01.2003
(51) Int. Cl.: A61L 9/20, F24C 15/20

(54) **ULTRAVIOLET LAMP VENTILATION APPARATUS AND METHOD**
VORRICHTUNG UND VERFAHREN FÜR ULTRAVIOLETT-VENTILATIONSLAMPEN
DISPOSITIF ET PROCEDE DE VENTILATION A LAMPES A U.V.

(30) Priority: 16.01.2002 US 349179 P
(43) Date of publication of application: 13.10.2004
(73) Proprietor: OY Halton Group Limited, 01510 Vantaa (FI)
(72) Inventor: GIBSON, Philip George, West Malling, Kent ME19 4PB (GB); HOBBS, Jeremy David, Warwickshire CV37 7JA (GB)
(74) Representative: Samuels, Adrian James
(86) International application number: PCT/GB2003/000122
(87) International publication number: WO 2003/061717

(56) References cited:
- EP-A- 1 134 782
- WO-A-00/78368
- WO-A-01/56624
- WO-A-97/34682
- WO-A-99/22777
- PATENT ABSTRACTS OF JAPAN vol. 018, no. 150 (C-1179), 14 March 1994 (1994-03-14) & JP 05 328897 A (MITSUBISHI HEAVY IND LTD;OTHERS: 01), 14 December 1993 (1993-12-14)

## Description

### FIELD OF THE INVENTION

This invention relates to a ventilation method and system for kitchens using ultra violet tubes or lamps: More particularly, the present invention relates to an ultra violet light assembly having a mode for regulating an amount of ozone incident to an air stream.

### BACKGROUND OF THE INVENTION

Ventilation systems are known for removing contaminated air produced by a cooking appliance. The contaminated air has contaminants, such as smoke, grease, odors and other unwanted airborne substances. Ultra Violet (UV) light is known to disinfect contaminants within a liquid or gas that is exposed thereto, with one example being contaminated air. UV light wavelengths commonly used for purification are about 185 nanometers (nm) and about 254 nm.

UV based air purification systems generally include a UV light source disposed to interact with a stream of contaminated air. UV light generally has a short wavelength. UV light in the C band has a wavelength in the range of about 180 nm to about 260 nm. The peak output of an ozone producing lamp is at a wavelength of about 253.7 nm. Generally, a drawback of UV lamps is that the UV lamps need time to stabilize and to operate at full output to assure germicidal properties.

The number of fast food businesses and catering establishments has risen in recent years. Over the same time period there has been established more stringent air pollution and odor pollution requirements and reported nuisances have also risen from these establishments. Contaminants released from these cooking establishments, especially from deep fat frying, result in fatty deposits and unfavorable odor. Furthermore, the potential for fire hazard due to a build up of grease and oil deposits is great. Typically, restaurateurs require costly high-pressure washing and maintenance in order to minimize the risk of fire hazard. There is a need to avoid the risk of a fire hazard and to reduce the amount of contaminants in the air stream expelled from establishments using a ventilation system.

Traditionally, commercial-catering establishments use mechanical filters to reduce contaminants. The use of High Efficiency Particulate Arresting (HEPA) filters to filter and collect particulate matter is well known in the art. Filters are not usually effective for ultra fine particles such as expected in food due to rapid clogging rates of the filter that also may result in a fire hazard. Also filters tend to collect grease and fat thereby creating fire hazards. Due to the nature of filter materials, there are also inherent limitations in temperature and humidity working conditions.

Another solution in the art has been catalytic oxidation of emissions to CO₂ and water. Oxidation of gaseous contaminants has the advantage that wastes are minimized. Catalytic oxidation requires electricity or high temperature ranges (250-350°C) for effectiveness. The catalysts also require a steady load of gaseous contaminants and a stable flow for the input in order to be effective. This method will not be very effective in commercial kitchens where the load of gaseous contaminants is not steady due to batch cooking and the temperatures are usually only up to 200°C.

Another solution in the art has been the use of activated carbon. Although the solution is effective there is a maintenance cost as absorption materials need to be changed regularly. Typically, this technology is based on the absorption properties of porous carbon material, which is used to arrest pollutants. The carbon material becomes polluted with use and must be replaced with new materials. A drawback is that the level of grease and fat generated that will be deposited on the materials. Increased deposits lead to increased maintenance costs and labor intensive requirements. It is estimated that the cost of this method in this application will be 40% more than that of filters.

Another solution in the art is the combustion of gaseous materials. Combustion is only suitable for a large unit where there is a constant and a steady supply of gaseous contaminants to bum. This will not be particularly suitable for commercial catering applications as it is hazardous, cumbersome and requires a high level of maintenance. It also poses a risk of a fire hazard that the food establishment seeks to minimize. This method also generates its own pungent odor.

In commercial catering, the mechanism of operation is based on a combination of sterilization and destruction of organic matter by ultraviolet radiation at certain wavelengths using ozone. At these wavelengths the ability of ozone to readily oxidize other elements reduces deposition of fat and grease in the ductwork. The approach has had success in a laboratory under a controlled environment, but the approach enjoyed only limited success in practice. This is attributed to an unstable flow in the application environment, where the gaseous contaminants vary in such a dynamic way that the prior art is not capable of responding fast enough. A barrier to effective deployment of UV and ozone against odor and pollution abatement is an inability to control the UV and ozone generation mechanisms. This prevents a rapid response to variable and dynamic loads of pollution that is typical of commercial batch catering environment.

WO 00/78368 discloses the preamble to claims 1 and 31 and shows an air purifier disposed in a return duct of an HVAC unit. The purifier produces at least two separate UV energy intensity maxima in distinct regions of the system. WO 99/22777 discloses an ozone chamber configured to reduce a through-flow velocity of an air stream.

JP-A-05328897 discloses a unit having a UV lamp, a catalyst and a case which covers the UV lamp.

Typically, restaurants and catering operations do not produce uniform amounts of contaminants throughout the course of a day. The amount of gaseous contaminants depends upon the food type, cooking process and the cooking load. At peak times of the day, optimum amounts of UV and ozone are desired. At the lowest point of activity, when there are reduced cooking loads, a minimum amount of UV and ozone is desired to avoid excess ozone production and the attendant ozone odor. Therefore, while the mechanism of odor control and the effect on gaseous contaminants is effective, the use of UV lamps is very complex and difficult to control, hence, the difficulties associated with the implementation in practice.

Ozone production incident to an air stream is effective on both gaseous contaminants and other biological contaminants that propagate odors. However, for this method to be used for pollution abatement, there is a need for it to be combined with an effective apparatus for regulating the UV and ozone incident upon an air stream that is disposed in the duct.

There is a need for an improved air purifier that dynamically regulates the concentration of ozone incident to an air stream and/or the time of such incidence.

There is a need for an improved air purifier that continually shortens response time for ozone to be incident to an air stream.

There is also a need for a safe, dynamic response to emission input loads by a multivariable controlled, integrated pollution and odor abatement system.

There is also a need for a system capable of removing solid and liquid matter as well as neutralizing gaseous contaminants and odors while preventing the build up of fat and grease that are potential fire hazard:

### SUMMARY OF THE INVENTION

According to first aspect of the present invention, an apparatus is provided as claimed in claim 1. An ultraviolet light source produces ozone incident upon an air stream in a ventilating duct. The apparatus also has a regulator that regulates the ozone incident upon the air stream. The regulator has a modulating structure that modulates the ozone and also has a control circuit. The control circuit preferably responds to signals corresponding to a level of contamination or a level of ozone of the air stream to control the modulating structure. The modulating structure may be a baffle, a catalyst, an intermediate member, or any combination thereof.

According to still another preferred embodiment of the present invention, the regulator has at least one sensor disposed in the ventilating duct, a microprocessor, and the modulating structure. The microprocessor responds to signals provided by at least one sensor to actuate the modulating structure in response to the contaminants. The modulating structure is actuated between a maximum ozone producing position corresponding to a maximum amount of ozone incident on the air stream and a minimum ozone producing position corresponding to a minimum amount of ozone incident on the air stream.

According to still yet another preferred embodiment of the present invention, the microprocessor controls an actuator for configuring the baffle into a first travel path corresponding to a maximum amount of ozone incident on the air stream and into a second travel path. The second travel path corresponds to a minimum amount of ozone on the air stream. The first travel path has a distance greater than the second travel path.

According to still another preferred embodiment of the present invention, the apparatus has a vibrator for vibrating a first and second electrostatic precipitators. This removes contamination and particulate matter from the first and second electrostatic precipitators.

According to another aspect of the present invention, there is provided a method of purifying an air stream as claimed in claim 31.

### BRIEF DESCRIPTION OF THE DRAWINGS

Other and further objects, advantages and preferred features of the present invention will be understood by reference to the following specification in conjunction with the accompanying drawings, in which like reference characters denote like elements of structure and:
FIG. 1 is a block diagram of an air purifier embodying the present invention.
FIG. 2 is a cross-sectional view of the air purifier of a preferred embodiment with an array of UV lamps and an array of baffles in a closed position.
FIG. 3 is a partial cross-sectional view of the air purifier of a preferred embodiment with the array of baffles in an opened position.
FIG. 4 is a cross-sectional view of another embodiment of the air purifier of the present invention with an array of UV lamps having a shield.
FIG. 5 is a cross-sectional view of still another embodiment of the air purifier of the present invention having an exemplary catalyst.
FIG. 6 is a cross-sectional view of still yet another embodiment of the air purifier of the present invention having an exemplary catalyst and an intermediate member disposed between the array of the UV lamps and the exemplary catalyst.
FIG. 7 is a cross-sectional view of still another further embodiment of the air purifier of the present invention having an exemplary catalyst and an intermediate member having one or more apertures disposed between the UV lamps and the exemplary catalyst.

### DESCRIPTION OF THE PREFERRED EMBODIMENT

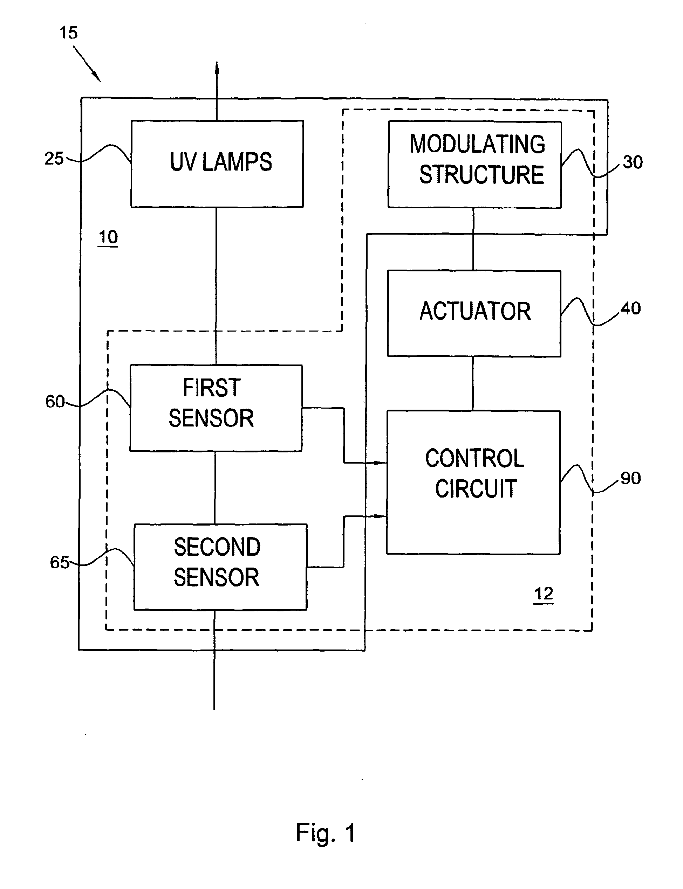

With reference to Fig. 1, there is provided a block diagram of an air purifier 15 in accordance with the present invention. Air purifier 15 may be disposed in a conventional commercial or residential ventilation duct 10 adjacent to a cooking source (not shown) as is known in the art. An air stream (shown by the arrow in Fig. 1) passes through the ventilating duct 10. Air purifier 15 includes UV lamps 25 that produce ozone to treat contaminants in the air stream and a regulator 12 that regulates the ozone incident to the air stream.

Regulator 12 is at least partially disposed in ventilation duct 10. Regulator 12 has a modulating structure 30, an actuator 40 preferably disposed outside of the ventilating duct 10, a first sensor 60, a second sensor 65 and a control circuit 90. The modulating structure 30 modulates the ozone incident upon the air stream and is controlled by control circuit 90. The control circuit 90 responds to signals provided by the first sensor 60 and the second sensor 65 corresponding to a level of contamination or ozone in the air stream, and controls the modulating structure 30 accordingly by actuator 40. Control circuit 90 preferably is a microprocessor, more preferably a DSP type processor or other suitable processor. Control circuit 90 monitors the output of the first sensor 60 and second sensor 65 to modulate the modulating structure 30 by actuator 40.

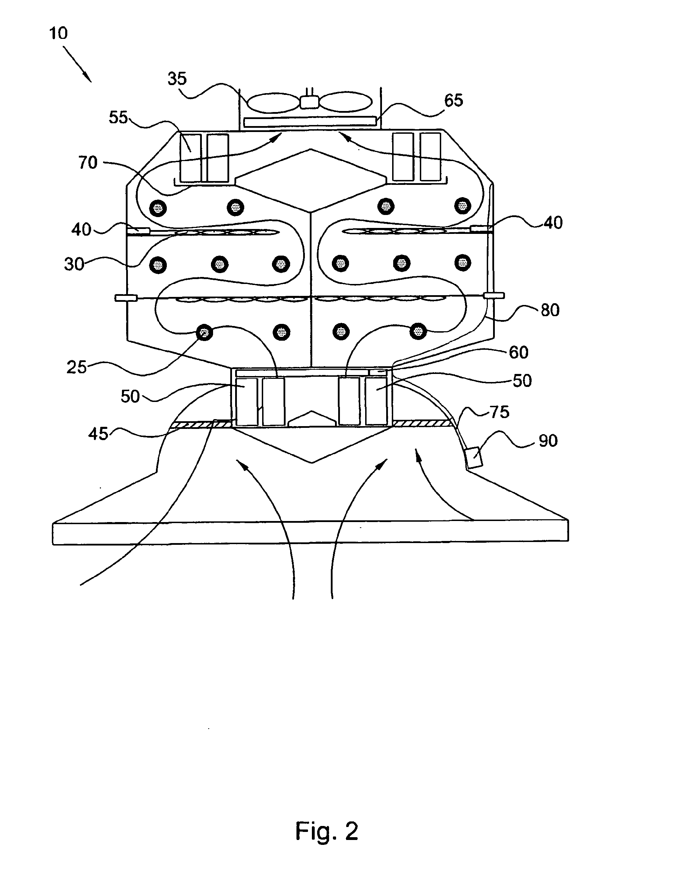

With reference to Fig. 2, UV lamps 25 comprise an array of ozone producing UV lamps 25 disposed in ventilation duct 10. Preferably, the UV lamps 25 are ultra violet light tubes that emit energetic photons and emit radiation having a wavelength less than about 200 nm. In another embodiment of the present invention, the UV lamps 25 emit radiation having a wavelength of about 185 nm wavelength. In another preferred embodiment, the UV lamps 25 have a wavelength of about 185 to 254 nm. Modulating structure 30 comprises an array of baffles 30. In addition, the ventilating duct 10 also has a fan 35, actuator 40, a removable metal pre-filter or coalescer 45, a plurality of first stage electrostatic precipitators 50 and a plurality of second stage electrostatic precipitators 55. Disposed at an inlet of the ventilation duct 10 is first sensor 60. Disposed near or at the outlet of the ventilation duct 10 is second sensor 65. The ventilation duct 10 also has a particulate collecting tray 70. Control circuit 90 controls the one or more operations of the UV lamps 25, the actuator 40 and any other operative functions is disposed at a suitable location located near the ventilation duct 10.

Control circuit 90 is also operatively connected to the actuator 40, UV lamps 25, first sensor 60 and second sensor 65, a fan 35 and a power source (not shown). One aspect of UV lamps 25 is that they emit photons in a wavelength range of about 185 nm. The photons emitted from the UV lamps 25 are sufficient to produce mono-atomic oxygen in a contaminated air stream emitted from the cooking source (not shown) in spaced relation to the UV lamps 25. UV lamps 25 are disposed substantially perpendicular to the flow of contaminated air passing through ventilating duct 10. An aspect of the UV lamps 25 is the rapid response of regulating the amount of ozone incident to an air stream.

UV lamps 25 are disposed in ventilating duct 10 in a series of rows, in a series of columns or in any suitable arrangement for emitting a germicidal dosage of ultra violet light in the contaminated air stream in the ventilating duct 10. Baffles 30 are disposed between the rows of the UV lamps 25. Baffles 30 are vertically disposed in the ventilating duct 10 in a suitable arrangement for selectively altering the flow of the contaminated air stream between the rows of the UV lamps 25. Baffles 30 may increase a travel path or pressure of the contaminated air stream around the UV lamps 25.

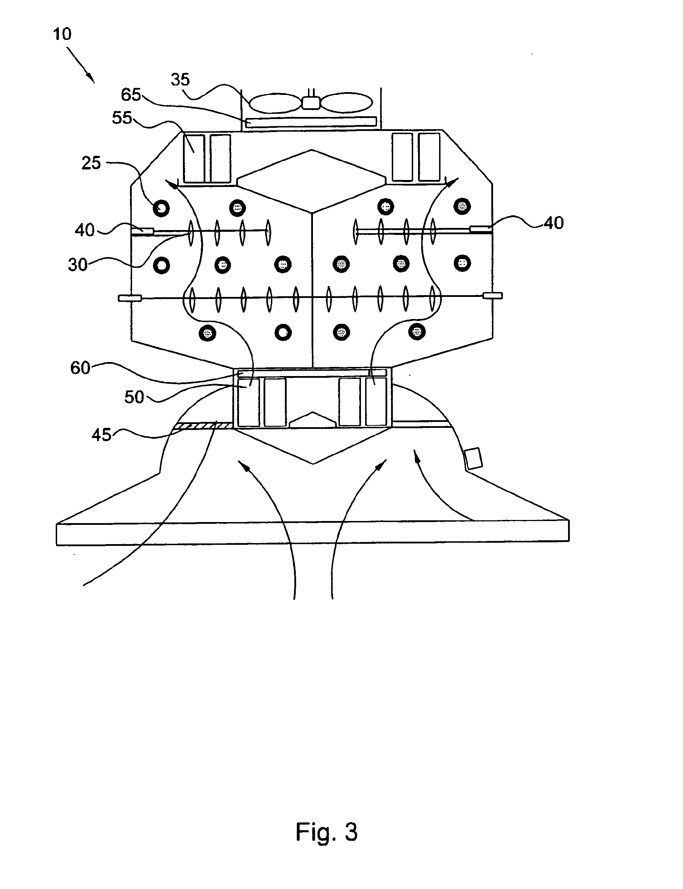

In one embodiment, the baffles 30 may have an opened position and a closed position. In the opened position shown in Fig. 3, the contaminated air stream may have a relatively shortened travel path, whereas in a closed position shown in Fig 2, the baffles may travel in a relatively longer travel path around the UV lamps 25 for increased exposure to the UV light. The array of baffles 30 selectively open or close by being actuated by the actuator 40.

Referring again to Figs. 2 and 3, as mentioned a contaminated air stream passes through ventilating duct 10 in the direction toward fan 35. In an exemplary embodiment of the present invention, when engaging in a light cooking load there is a reduced amount of oil and grease in the contaminated air stream. Here, baffles 30 are disposed in the open position as shown in Fig. 3. This opened position allows the contaminated air stream to pass through a relatively short travel or relatively lower pressure path in the ventilating duct 10.

However, during a high cooking load, the contaminated air stream has an increased amount of oil, grease and contaminants. A first sensor 60 in response thereto communicates a first signal to actuator 40 for actuating one or more of the array of baffles 30 to the closed position. The closed position increases the travel path or increases the pressure of the contaminated air stream for increasing the exposure time of the UV lamps 25 to the contaminated air stream as shown in Fig. 2.

First sensor 60 may be a flame ionization sensor, a photo-ionization sensor, an infrared sensor, a gas chromatography, mass spectrometry sensor, a bulk wave acoustic sensor, a surface wave acoustic sensor, a metal oxide based sensor or any other sensor known in the art. First sensor 60 communicates a first signal that corresponds to either a high cooking load or a second signal corresponding to a low cooking load to control circuit 90.

In an alternative embodiment, second sensor 65 also may communicate either a first signal that corresponds to either a high cooking load or a second signal corresponding to a low cooking load to control circuit 90. In still another embodiment, second sensor 65 may either communicate a first signal that corresponds to either a high concentration of ozone or a second signal corresponding to a low concentration of ozone to control circuit 90 for regulating ozone. Referring to Figs. 2 and 3, the second sensor 65 preferably measures the amount of ozone at the end opposite the cooking source that is disposed beneath the ventilation duct 10 and the first sensor 60 preferably measure the amount of contaminants. Here, first sensor 60 communicates a first signal and second sensor 65 communicates a second signal to control circuit 90 for controlling UV lamps 25 and/or baffles 30.

An example of first sensor 60 and second sensor 65 may be a surface acoustic wave chemical sensor in a matrix with a pressure transducer (not shown). Pressure transducer senses the level of loading while the matrix of the acoustic wave chemical sensor senses a variety of polar (such as alcohol esters and ketones) and non-polar oxygenated nitrogenated and chlorinated compounds. In one exemplary embodiment of the present invention, first sensor 60 and second sensor 65 may be a polar compound sensor that senses largely polar organic compounds on the input while sensing oxygenated matter or ozone at the output.

An aspect of first sensor 60 and second sensor 65 is that the sensors are suitable for measurement, preferably in milliseconds. Another aspect of first sensor 60 and second sensor 65 is that first sensor 60 and second sensor 65 are accurate without regular maintenance and are easily integrated with the control circuit 90 for a cost-effective installation.

In one exemplary embodiment of the present invention, the array of UV lamps 25 synchronized by a linkage will be used in the ventilation duct 10 for regulating dynamically the ozone concentration in the system based on a feedback loop in control circuit 90.

Since first sensor 60 and second sensor 65 can be affected by the continuous presence of dust, a suitable shielding system (not shown) is provided for preferably keeping out dust, grease and other contaminants. First sensor 60 is placed behind the first stage electrostatic precipitators 50 to protect second sensor 65 from dust and prevent an erroneous reading of the level of gaseous contaminants.

In response to a high cooking load, actuator 40 may shift baffles 30 from a closed position to an opened position to decrease the travel path of the air stream, thereby decreasing the amount of ozone incident to the air stream. In another embodiment, actuator 40 may completely open only some of the baffles 30 while not opening the remainder. In still another embodiment of the present invention, actuator 40 may only slightly open or may only slightly close the baffles 30 for modulating the amount of ozone incident to the air stream.

In an embodiment of the present invention, actuator 40 directs the air stream to a first travel path, a second travel path or any combinations thereof. In response to a low cooking load, actuator 40 shifts the baffles 30 from a closed position to an opened position, thereby decreasing the amount of ozone incident to the air stream.

Actuator 40 comprises a motor, (not shown) hydraulic jack or manual actuator to actuate one or more baffles 30 from the open position to the closed position. In this embodiment, baffles 30 may increase the air pressure of the contaminated air stream that is passing through the ventilating duct 10. Air pressure is increased and contaminated air is forced to circulate around baffles 30 and in proximity to UV lamps 25 for increasing the exposure time of the UV lamps 25 to the contaminated air stream.

While the presence of ozone can convert gaseous contaminants into harmless by-products and sterilize and destroy bacterial causing odor, the abatement of particulate matters needs to be addressed. First and second electrostatic precipitators 50 and 55 preferably operate using a programmable switch mode power supply (not shown). The programmable switch mode power supply enables the user to use a vibrator (not shown) to vibrate a pole of the first and second electrostatic precipitators 50 and 55 so that all the contaminants can drop on to a collection tray 70 for disposal as a dry waste.

Actuator 40 is any suitable mechanical actuating mechanism, or other such suitable automatic or manual mechanical device, such as one connected to, for example a hydraulic jack (not shown) by a sensor link for actuating the array of baffles 30 in the contaminated air stream. Actuator 40 may have a series of gears (not shown) operatively connected to a motor (not shown) and may be bolted or fastened to ventilating duct 10 on an interior side of the ventilating duct. Baffles 30 and UV lamps 25 may be fastened to ventilating duct 10 by any suitable fastener, presently known or known in the future.

The present embodiment regulates the ozone incident upon an air stream. The regulator 12 has a modulating structure 30 that modulates the ozone incident on the air stream. The regulator 12 has control circuit 90 and the actuator 40. The control circuit 90 responds to signals corresponding to a level of contamination of the air stream to control and impart a modulating motion to the modulating structure.

An exemplary aspect of the present invention is the ability to regulate the ultra violet light emitted by UV lamps 25 thereby modulating an amount of ozone that is incident to the contaminated air stream. By modulating the amount of ozone, a user optimally provides for a desired amount of germicidal properties to the contaminated air stream while simultaneously minimizing any possible ozone odor depending upon an amount of cooking emissions released from the cooking source.

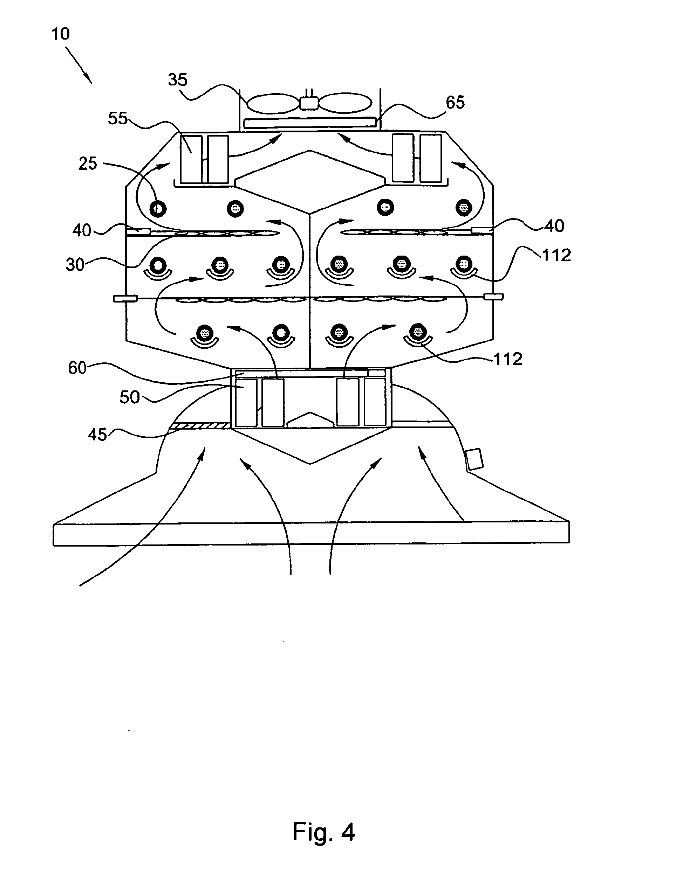

With reference to Fig. 4, in another embodiment of modulating the amount of ozone incident to the contaminated air stream a shield 112 is provided. Shield 112 may be any suitable shape or size for shielding the UV lamps from any cooling effect of the contaminated air stream.

Shield 112 may be U shaped, T shaped, V shaped, W shaped, arcuately shaped, parabolic, oblong or any other shape or size that can be disposed on the upstream side of UV lamps 25 maintaining a temperature of the UV lamps. Shield 112 preferably prevents the UV lamps 25 from cooling. Shield 112 shields air flow in front of the UV lamps 25, to prevent the contaminated air stream from cooling the UV lamps 25 thereby maintaining an intensity of the UV lamps and increasing the amount of ozone incident to the contaminated air stream.

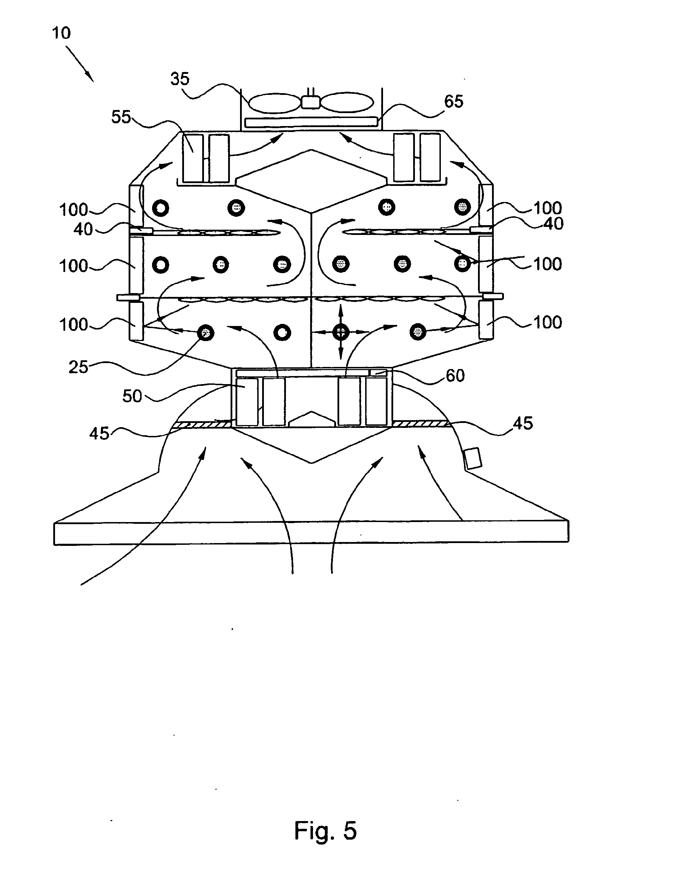

With reference to Fig. 5, another embodiment of regulating the amount of ozone incident to the contaminated air stream provides a catalyst 100 that is located at a predetermined distance from the UV lamps 25 so as to intensify the reaction of UV light illuminated on the passing air stream. In one embodiment, at least one or the UV lamps 25 may have a catalyst 100 operatively connected to the actuator 40. Actuator 40 may further comprises a first gear, a second gear, a motor and an output shaft for moving the catalyst 100 or the UV lamps 25 relative to the other. As mentioned, in another embodiment, actuator 40 is a jack or motor preferably having an input and output coil (not shown) that is electrically connected to power source (not shown) is used for actuating the catalyst 100 or the UV lamps 25 relative to the other.

Catalyst 100 may be a substantially cylindrical structure, a semispherical structure, a polyhedron, a rectangular member, a pyramid shaped member, an oblong structure, a rectangular structure, a spherical structure or a walled surface in an interior of the ventilating duct 10 as illustrated in Fig. 5. Catalyst 100 may also be disposed on at least a portion of the shield 112 or may be any shape for effectively reflecting an amount of germicidal ultra-violet light from catalyst to create ozone incident to a contaminated air stream.

Catalyst 100 may also be disposed more distant from the UV lamps 25 one or more walls, on one or more baffles, housing, floors, or structures in the ventilation duct 10. Alternatively, catalyst 100 may also be a suitably shaped member disposed in concentric relation over UV lamps 25.

It is not practical to constantly switch on and off UV lamps 25 as the life of the UV lamps will be significantly reduced. This will result in increased maintenance costs to the food establishment resulting in constant replacement of the UV lamps 25 and service costs associated with the replacement. Therefore, in this embodiment, it is preferable that UV lamps 25 be left in the illuminated position to obviate the start up time to reach optimum performance. It has been shown that the ratings of the UV lamps 25 indicate that the ozone generation is at a concentration of approximately 0.9mg/m³ based on the air volume of the ventilation duct 10 but the maximum ozone generation preferably is 0.16-0.18 mg/m³.

Catalyst 100 is any suitable material for reflecting germicidal UV light and preferably is a titanium dioxide material, a material coated with titanium oxide or any other reflective material or reflective coating. Preferably, as shown in Fig. 5, the UV lamps 25 are exposed to the coated titanium dioxide material in a first position for a maximum ozone generation. The UV lamps are moved away from the catalyst 100 to at least a second position for minimum ozone generation. When UV lamps 25 are spaced closer to catalyst 100, an increased amount of UV light is exposed to the contaminated air stream and, accordingly, an increased amount of ozone is incident to the contaminated air stream. When the sensors indicate that less ozone is needed, UV lamps 25 will be spaced further from the catalyst 100.

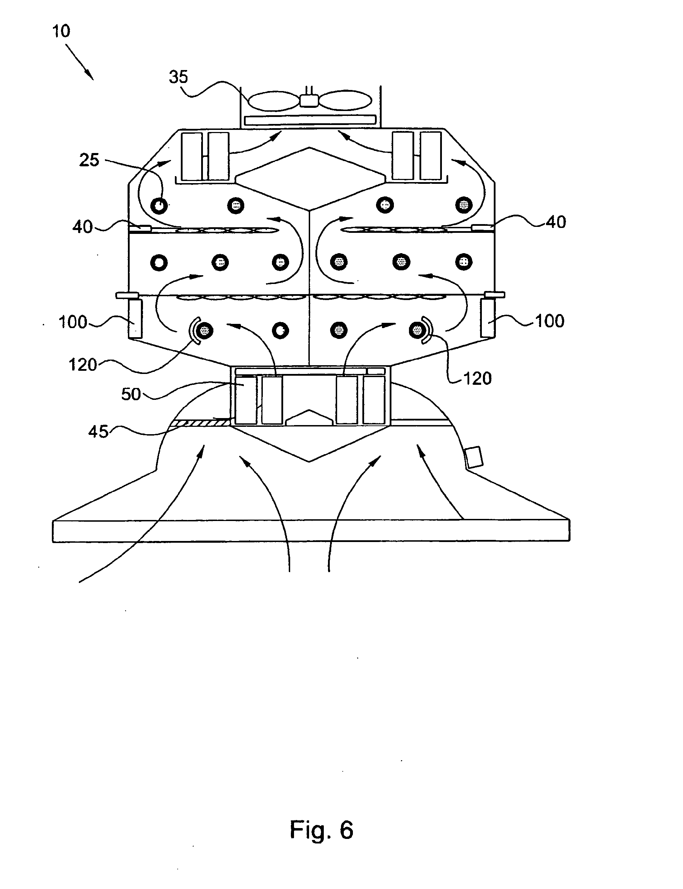

Referring to Fig. 6, another embodiment of the present invention regulates the amount of ozone incident to an air stream by providing UV lamps 25 with an intermediate member 120. Intermediate member 120 is any non-reflective solid material having a predetermined surface area suitable for blocking or otherwise covering catalyst 100 or covering the UV lamps 25. When UV lamps 25 are blocked from the catalyst 100 by the non-reflective intermediate member 120, a decreased amount of UV light is exposed to the contaminated air stream relative to the instance when UV lamp 25 is exposed to the catalyst 100. Accordingly, a relatively decreased amount of ozone is produced incident to the contaminated air stream. This preferred embodiment results in less ozone incident to the contaminated air stream.

An exemplary aspect of the present invention, is that in one preferred embodiment, intermediate member 120 is operatively connected to actuator 40. In this manner, actuator 40 rotates catalyst 100 closer to, or farther away from the UV lamps 25. Actuator 40 may also rotate intermediate member 120 to block the catalyst 100 from UV lamps 25.

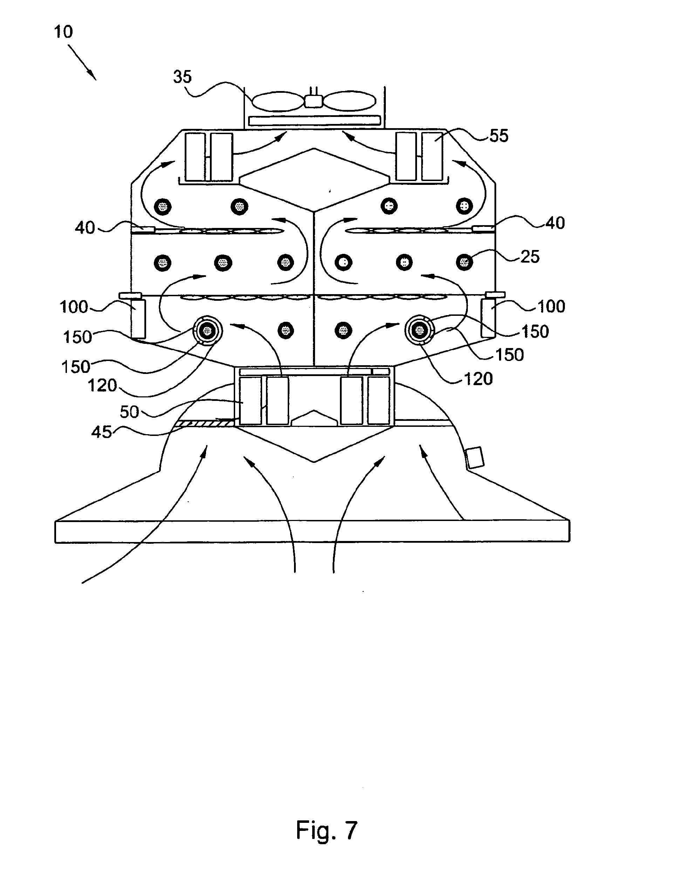

Another embodiment of the present invention shown as Fig. 7 provides intermediate member 120 having one or more apertures 150. The apertures 150 allow intermediate member 120 to selectively expose or cover the catalyst 100 with minimum rotation of intermediate member 120. This selective blocking maintains or decreases the exposure of UV lamps 25 to catalyst 100 and the contaminated air stream, thereby allowing a user to control the intensity of the UV lamps 25 without completely terminating the illumination of the UV lamps 25. Apertures 150 are disposed on intermediate member 120 in a suitable pattern and may have any shape or size. Intermediate member 120 selectively shields or otherwise blocks UV lamps 25 from catalyst 100 by a minimal rotation of the intermediate member 120 to vary exposure of the UV lamp 25 to catalyst 100 and the contaminated air stream.

In another preferred embodiment of the present invention, (not shown) one or more UV lamps 25 are moved away from catalyst 100 by actuator 40 thereby reducing the amount of UV light exposed to the contaminated air stream resulting in less ozone incident to the contaminated air stream. Similarly, UV lamps 25 are moved toward a stationary catalyst 100 by actuator 40. Catalyst 100 may also be formed as a circular shaped member with a diameter sufficient to envelop multiple UV lamps 25.

Ventilating duct 10 may be any suitable ventilating duct known. Excess contaminants, such as grease, flow down removable metal pre-filter 45 and collect in gutter (not shown) for subsequent removal by service personnel and to minimize fire hazard. It should be apparent to one skilled in the art, that the present invention may be used either in commercial or residential ventilating ducts or any other air purifier utilizing UV lamps.

Contaminated airflows from a cooking source (not shown) through a removable pre-filter 45. Removable pre-filter 45 prevents oil and grease droplets to pass through pre-filter 45 and instead causes oil and grease to flow opposite the UV lamps 25 to maintain the operational performance the UV lamps and for safety reasons.

The present invention having been thus described with particular reference to the preferred forms thereof, it will be obvious that various changes and modifications may be made therein without departing from the scope of the present invention as defined in the appended claims.

## Claims

1. An apparatus comprising:
a ultraviolet light source (25) which is capable of producing ozone incident upon an air stream in a ventilating duct (10); and
a regulator (12) that regulates said ozone incident upon said air stream, said regulator (12) having a control circuit (90) operatively connected to said ultraviolet light source (25), wherein said control circuit (90) responds to a first signal corresponding to a level of contamination of said air stream or a second signal corresponding to a level of ozone in said air stream and regulates an illumination of said ultraviolet light source (25), said control circuit (90) regulating said ozone incident said air stream by regulating power from said power supply to said ultraviolet light source, and **characterised in that** said regulator comprises a modulating structure (30; 100; 112; 120) that modulates said ozone incident said air stream and said control circuit is also operatively connected to said modulating structure.

2. The apparatus of claim 1, wherein said control circuit (90) responds to a plurality of signals corresponding to a level of contamination or a level of ozone of said air stream to control said modulating structure (30).

3. The apparatus of claim 1, further comprising an actuator (40) for actuating said modulating structure (30) for modulating said ozone.

4. The apparatus of claim 2, wherein said regulator (12) further comprises an actuator (40), and wherein said control circuit (90) responds to signals corresponding to said level of ozone or contamination of said air stream to control said actuator (40) to impart a modulating motion to said modulating structure (30).

5. The apparatus of claim 3 or 4, wherein said regulator (12) further comprises one or more sensors (60, 65) that provide said plurality of signals.

6. The apparatus of claim 3, 4 or 5, wherein said signals are provided by a first sensor (60) and a second sensor (65) respectively corresponding to said level of contamination and said level of ozone in said ventilating duct (10).

7. The apparatus of any preceding claim, wherein said control circuit (90) further regulates said ozone by actuating said modulating structure (30) to a maximum position corresponding to a maximum amount of ozone incident upon said air stream and a minimum position corresponding to a minimum amount of ozone incident on said air stream.

8. The apparatus of any preceding claim, wherein said modulating structure is selected from the group consisting of: a baffle (30), a catalyst (100), a shield (112), an intermediate member (120), and any combination thereof.

9. The apparatus of any preceding claim, wherein said modulating structure (30) comprises one or more baffles (30) disposed in an arrangement for regulating a travel path of said air stream that is incident to said ozone.

10. The apparatus of any preceding claim, wherein said modulating structure (30) further comprises a plurality of baffles (30) disposed in said ventilating duct (10) in an array, and wherein said plurality of baffles (30) open and close to vary a distance of a travel path of said air stream that is incident to said ozone.

11. The apparatus of any preceding claim, wherein said modulating structure (30) comprises a plurality of baffles (30) that are configurable into at least two travel paths of different distances for regulating said ozone.

12. The apparatus of any of claims 1-8, wherein said modulating structure (30) comprises a catalyst (100) that is disposed in said ventilating duct (10), and wherein said regulator (12) varies an exposure of said ultraviolet light source (25) to said catalyst (100) so as to regulate an amount of said ozone.

13. The apparatus of claim 12, wherein said catalyst (100) is selected from the group consisting of: titanium dioxide, a ultraviolet light source reflective material that increases the amount of ozone produced by said ultraviolet light source (25), and any combination thereof.

14. The apparatus of claim 12 or 13, wherein said catalyst (100) is movable between a maximum ozone producing position and a minimum ozone producing position that is more distant from said ultraviolet light source (25) than said maximum ozone producing position.

15. The apparatus of claim 12, 13 or 14, wherein said regutator (12) moves said catalyst (100) and/or said ultraviolet light source (25) relative to one another to vary said exposure of said ultraviolet light source (25) to said catalyst (100) for regulating said ozone incident on said air stream.

16. The apparatus of any of claims 1-8, wherein said modulating structure (30) comprises an intermediate member (120) disposed between said ultraviolet light source (25) and said air stream.

17. The apparatus of claim 16, wherein said intermediate member (120) has at least one aperture (150).

18. The apparatus of claim 16 or 17, wherein said intermediate member (120) has a plurality of apertures (150) disposed in a pattern.

19. The apparatus of claim 16, 17 or 18, wherein said modulating structure (30) comprises an intermediate member (120) disposed between said ultraviolet light source (25) and said air stream, said intermediate member (120) being movable between an ozone producing maximum position to an ozone producing minimum position.

20. The apparatus of any of claims 1-8, wherein said modulating structure (30) comprises a shield (112) disposed between said ultraviolet light source (25) and said air stream, said shield maintaining a temperature of said ultraviolet light source (25).

21. The apparatus of claim 20, wherein said shield (112) is disposed upstream of said ultraviolet light source (25).

22. The apparatus of any preceding claim, wherein said control circuit (90) regulates said ozone incident upon said air stream by toggling said ultraviolet light source (25) to a state selected from the group consisting of: an illuminated state, a non illuminated state, a reduced illuminated state relative to said illuminated state, and any combination thereof.

23. The apparatus of any preceding claim, wherein said regulator (12) further comprises a fan (35) to regulate a velocity of said air stream passing over said ultraviolet light source (25).

24. The apparatus of any preceding claim, wherein said ultraviolet light source (25) is an ultra violet light tube that emits energetic photons.

25. The apparatus of any preceding claim, wherein said ultraviolet light source (25) emits radiation having a wavelength in the range between about 185 nm to 254 nm wavelength.

26. The apparatus of any of claims 1-24, wherein said ultraviolet light source (25) emits radiation having a wavelength less than about 200 nm.

27. The apparatus of claim 1, wherein said regulator (12) further comprises at least one sensor (60, 65) disposed in said ventilating duct (10), a microprocessor (90) and a modulating structure (30), wherein said modulating structure (30) is one selected from the group consisting of: a baffle (30), a catalyst (100), an intermediate member (120), and any combination thereof.

28. The apparatus of claim 27, wherein said microprocessor (90) responds to signals provided by said at least one sensor (60, 65) for actuating said modulating structure (30), in response to said contaminants, from an maximum ozone producing position corresponding to a maximum amount of ozone incident on said air stream to a minimum ozone producing position corresponding to a minimum amount of ozone incident on said air stream.

29. The apparatus of claim 27 or 28, wherein said modulating structure (30) is a baffle (30), and wherein said microprocessor (90) controls an actuator (40) for controlling said baffle (30) forming a first travel path corresponding to a maximum amount of ozone incident on said air stream and forming a second travel path corresponding to a minimum amount of ozone on said air stream, wherein said first travel path has a distance greater than said second travel path.

30. The apparatus of any preceding claim, further comprising a vibrator for vibrating a first (50) and a second (55) electrostatic precipitators for removing contamination and/or particulate matter from said first (50) and second (55) electrostatic precipitators.

31. A method of purifying an air stream, comprising:
(a) passing the air stream by an ultraviolet light source (25) sufficient to create ozone in the air stream ; and
(b) regulating an amount of said ozone incident upon the air stream by determining a level of contaminants or a level of ozone in the air stream and regulating an illumination of said ultraviolet light source (25) in response to said amount of contaminants and said amount of ozone by regulating power from a power supply to said ultraviolet light source, and **characterised by** said regulating step including modulating said ozone incident the air stream, wherein said modulating is affected by a modulating structure (30) that modulates said ozone incident upon the air stream.

32. The method of claim 31, further comprising the step of detecting said level of contamination of the air stream, and said modulating is effected by moving said modulating structure (30) in response to said level of contamination.

33. The method of claim 31, further comprising the step of detecting said level of ozone of the air stream, and said modulating is effected by moving said modulating structure (30) in response to said level of ozone.

34. The method of claim 31, wherein said modulating is effected by a modulating structure (30) selected from the group consisting of: a baffle (30), a catalyst (100), a shield (112), an intermediate member (120) and any combination thereof.

35. The method of any of claims 31-34, further comprising the step of regulating a travel path of said air stream.

36. The method of claim 31, wherein said modulating structure (30) further comprises a catalyst (100), and wherein the method further has the step of regulating a distance of said catalyst (100), and said ultra violet light source (25) relative to one another.

37. The method of claim 31, wherein said modulating structure (30) further comprises a catalyst (100) and an intermediate member (120), disposed between said ultra violet light source (25) and said catalyst (100), and wherein the method further has the step of moving said intermediate member (120) to vary radiation emitted by said ultra violet light source (25) that is incident upon said catalyst (100).

38. The method of any of claims 31-37, wherein step (b) regulates power to said ultra violet light source (25).

39. The method of any of claims 31-38, wherein step (b) regulates a velocity of the air stream.

40. The method of any of claims 31-39, wherein said ultra violet light source (25) is an ultra violet light tube that emits energetic photons.

41. The method of any of claims 31-40, wherein said ultra violet light source (25) emits radiation having a wavelength that is less than about 200 nm.

42. The method of any of claims 31-40, wherein said wavelength is in a range from about 185 nm wavelength to 254 nm wavelength.

43. The method of claim 31, 32 or 33, wherein said modulating structure (30) comprises a baffle arrangement (30) disposed in a ventilating duct (10), and wherein the method further has the step of controlling said baffle arrangement (30) to vary the distance of a travel path of the air stream.

44. The method of claim 31, wherein said modulating structure (30) is selected from the group consisting of: a baffle (30), a catalyst (100), an intermediate member (120), and any combinations thereof.

45. The method of claim 44, wherein said catalyst (100) is selected from the group consisting of: titanium dioxide, a ultra violet light reflective material that increases the amount of ozone produced by said ultra violet light source (25) and any combinations thereof.

46. The method of claim 44, wherein said baffle (30) is disposed in an array, and wherein said baffle (30) opens and closes to vary a distance of a travel path of the air stream that is incident to said ozone.

47. The method of claim 45, wherein said catalyst (100) varies an exposure of said ultra violet light source (25) to regulate an amount of ozone incident on the air stream.

48. The method of claim 31, further comprising the step of moving a catalyst (100) and/or said ultra violet light source (25) relative to one another to vary an exposure of said ultra violet light source (25) to said catalyst (100) for regulating said ozone incident on the air stream.

49. The method of claim 48, further comprising the step of placing an intermediate member (120) between said catalyst (100) and said ultra violet light source (25), said intermediate member (120) being movable between an ozone producing maximum position to an ozone producing minimum position.

50. The method of any of claims 31-49, further comprising the step of shielding said ultra violet light source (25) from said air stream for at least maintaining a temperature of said ultra violet light source (25).

51. The method of claim 44, further comprising the step of actuating said modulating structure (30) in response to signals to form a maximum ozone producing position, and to form a minimum ozone producing position.

52. The method of claim 51, wherein said signals are provided by a first sensor (60) and a second sensor (65).

## Patentansprüche

1. Vorrichtung, die Folgendes umfasst:
eine Ultraviolettlichtquelle (25), die Ozon erzeugen kann, das in einem Lüftungskanal (10) auf einen Luftstrom fällt, und
einen Regler (12), der das auf den Luftstrom fallende Ozon reguliert, wobei der Regler (12) eine SteuerSchaltung (90) aufweist, die mit der Ultraviolettlichtquelle (25) wirkverbunden ist und auf ein erstes Signal reagiert, das einem Verschmutzungspegel des Luftstroms entspricht, oder auf ein zweites Signal, das einem Ozonpegel in dem Luftstrom entspricht, und eine Beleuchtung der Ultraviolettlichtquelle (25) reguliert, wobei die Steuerschaltung (90) das auf den Luftstrom fallende Ozon reguliert, indem sie den Strom von der Stromversorgung für die Ultraviolettlichtquelle reguliert, und **dadurch gekennzeichnet, dass**
der Regler eine Modulierstruktur (30, 100, 112, 120) aufweist, die das auf den Luftstrom fallende Ozon moduliert, und die Steuerschaltung auch mit der Modulierstruktur wirkverbunden ist.

2. Vorrichtung nach Anspruch 1, bei der die Steuerschaltung (90) auf mehrere Signale reagiert, die einem Verschmutzungspegel oder einem Ozonpegel des Luftstroms entsprechen, und die Modulierstruktur (30) steuert.

3. Vorrichtung nach Anspruch 1, die des Weiteren ein Stellglied (40) für das Betätigen der das Ozon modulierenden Modulierstruktur umfasst.

4. Vorrichtung nach Anspruch 2, bei der der Regler (12) des Weiteren ein Stellglied (40) umfasst und die Steuerschaltung (90) auf Signale reagiert, die dem Ozonpegel oder dem Verschmutzungspegel des Luftstroms entsprechen, und das Stellglied (40) so steuert, dass eine Modulierbewegung an die Modulierstruktur (30) vermittelt wird.

5. Vorrichtung nach Anspruch 3 oder 4, bei der der Regler (12) des Weiteren einen oder mehrere Sensoren (60, 65) umfasst, die die mehreren Signale bereitstellen.

6. Vorrichtung nach Anspruch 3, 4 oder 5, bei der die Signale dem Verschmutzungspegel beziehungsweise dem Ozonpegel in dem Lüftungskanal (10) entsprechend von einem ersten Sensor (60) beziehungsweise einem zweiten Sensor (65) bereitgestellt werden.

7. Vorrichtung nach einem der vorhergehenden Ansprüche, bei der die Steuerschaltung (90) das Ozon des Weiteren dadurch reguliert, dass sie die Modulierstruktur (30) in eine Maximalposition bringt, die einer Höchstmenge an auf den Luftstrom fallendem Ozon entspricht, und in eine Minimalposition, die einer Mindestmenge an auf den Luftstrom fallendem Ozon entspricht.

8. Vorrichtung nach einem der vorhergehenden Ansprüche, bei der die Modulierstruktur aus einer Gruppe ausgewählt wird, die aus einem Leitblech (30), einem Katalysator (100), einer Abschirmung (112), einem Zwischenelement (120) und einer beliebigen Kombination davon besteht.

9. Vorrichtung nach einem der vorhergehenden Ansprüche, bei der die Modulierstruktur (30) ein oder mehrere Leitbleche (30) umfasst, die so angeordnet sind, dass sie eine Bewegungsbahn des auf das Ozon fallenden Luftstroms regulieren.

10. Vorrichtung nach einem der vorhergehenden Ansprüche, bei der die Modulierstruktur (30) des Weiteren mehrere in dem Lüftungskanal (10) angeordnete Leitbleche (30) umfasst und sich diese mehreren Leitbleche (30) so öffnen und schließen lassen, dass sie die Länge einer Bewegungsbahn des auf das Ozon fallenden Luftstroms ändern.

11. Vorrichtung nach einem der vorhergehenden Ansprüche, bei der die Modulierstruktur (30) mehrere Leitbleche (30) umfasst, die sich zum Regulieren des Ozons in mindestens zwei Bewegungsbahnen von unterschiedlicher Länge konfigurieren lassen.

12. Vorrichtung nach einem der Ansprüche 1-8, bei der die Modulierstruktur (30) einen Katalysator (100) umfasst, der in dem Lüftungskanal (10) angeordnet ist, und der Regler (12) den Einfluss des Katalysators (100) auf die Ultraviolettlichtquelle (25) ändert, um eine Menge des Ozons zu regulieren.

13. Vorrichtung nach Anspruch 12, bei der der Katalysator (100) aus einer Gruppe ausgewählt wird, die aus Titandioxid, einem Ultraviolettlicht reflektierenden Material, das die von der Ultraviolettlichtquelle (25) erzeugte Ozonmenge erhöht, und einer beliebigen Kombination davon besteht.

14. Vorrichtung nach Anspruch 12 oder 13, bei der der Katalysator (100) zwischen einer Position für die maximale Ozonerzeugung und einer Position für die minimale Ozonerzeugung, die weiter von der Ultraviolettlichtquelle (25) entfernt ist als die Position für die maximale Ozonerzeugung, verschoben werden kann.

15. Vorrichtung nach Anspruch 12, 13 oder 14, bei der der Regler (12) den Katalysator (100) und/ oder die Ultraviolettlichtquelle (25) in Bezug zueinander verschiebt, um den Einfluss des Katalysators (100) auf die Ultraviolettlichtquelle (25) zu ändern und das auf den Luftstrom fallende Ozon zu regulieren.

16. Vorrichtung nach einem der Ansprüche 1-8, bei der die Modulierstruktur (30) ein Zwischenelement (120) umfasst, das zwischen der Ultraviolettlichtquelle (25) und dem Luftstrom angeordnet ist.

17. Vorrichtung nach Anspruch 16, bei der das Zwischenelement (120) mindestens eine Öffnung (150) aufweist.

18. Vorrichtung nach Anspruch 16 oder 17, bei der das Zwischenelement (120) mehrere Öffnungen (150) aufweist, die in einem Muster angeordnet sind.

19. Vorrichtung nach Anspruch 16, 17 oder 18, bei der die Modulierstruktur (30) ein Zwischenelement (120) umfasst, das zwischen der Ultraviolettlichtquelle (25) und dem Luftstrom angeordnet ist, wobei sich das Zwischenelement (120) zwischen einer Position für die maximale Ozonerzeugung und einer Position für die minimale Ozonerzeugung verschieben lässt.

20. Vorrichtung nach einem der Ansprüche 1-8, bei der die Modulierstruktur (30) eine Abschirmung (112) umfasst, die zwischen der Ultraviolettlichtquelle (25) und dem Luftstrom angeordnet ist, wobei die Abschirmung eine Temperatur der Ultraviolettlichtquelle (25) aufrechterhält.

21. Vorrichtung nach Anspruch 21, bei der die Abschirmung (112) vor der Ultraviolettlichtquelle (25) angeordnet ist.

22. Vorrichtung nach einem der vorhergehenden Ansprüche, bei der die Steuerschaltung (90) das auf den Luftstrom fallende Ozon reguliert, indem sie die Ultraviolettlichtquelle (25) in einen Zustand umschaltet, der aus der Gruppe ausgewählt wird, die aus einem beleuchteten Zustand, einem nicht beleuchteten Zustand, einem Zustand mit im Vergleich zum beleuchteten Zustand verringerter Beleuchtung und einer beliebigen Kombination davon besteht.

23. Vorrichtung nach einem der vorhergehenden Ansprüche, bei der der Regler (12) des Weiteren einen Ventilator (35) umfasst, der die Geschwindigkeit des über die Ultraviolettlichtquelle (25) strömenden Luftstroms reguliert.

24. Vorrichtung nach einem der vorhergehenden Ansprüche, bei der es sich bei der Ultraviolettlichtquelle (25) um eine Ultraviolettlampe handelt, die energiereiche Photonen emittiert.

25. Vorrichtung nach einem der vorhergehenden Ansprüche, bei der die Ultraviolettlichtquelle (25) Strahlung mit einer Wellenlänge im Bereich von ungefähr 185 nm bis 254 nm emittiert.

26. Vorrichtung nach einem der Ansprüche 1-24, bei der die Ultraviolettlichtquelle (25) Strahlung mit einer geringeren Wellenlänge als ungefähr 200 nm emittiert.

27. Vorrichtung nach Anspruch 1, bei der der Regler (12) des Weiteren mindestens einen Sensor (60, 65), der in dem Lüftungskanal (10) angeordnet ist, einen Mikroprozessor (90) und eine Modulierstruktur (30) umfasst,
wobei die Modulierstruktur (30) aus einer Gruppe ausgewählt wird, die aus einem Leitblech (30), einem Katalysator (100), einem Zwischenelement (120) und einer beliebigen Kombination davon besteht.

28. Vorrichtung nach Anspruch 27, bei der der Mikroprozessor (90) auf Signale reagiert, die als Reaktion auf die Verschmutzungen von dem mindestens einen Sensor (60, 65) bereitgestellt werden, damit die Modulierstruktur (30) von einer Position für die maximale Ozonerzeugung, die einer Höchstmenge an auf den Luftstrom fallendem Ozon entspricht, in eine Position für die minimale Ozonerzeugung, die einer Mindestmenge an auf den Luftstrom fallendem Ozon entspricht, gebracht wird.

29. Vorrichtung nach Anspruch 27 oder 28, bei der es sich bei der Modulierstruktur (30) um ein Leitblech (30) handelt und der Mikroprozessor (90) ein Stellglied (40) steuert, das das Leitblech (30) steuert, das eine erste Bewegungsbahn bildet, die einer Höchstmenge an auf den Luftstrom fallendem Ozon entspricht, und eine zweite Bewegungsbahn, die einer Mindestmenge an auf den Luftstrom fallendem Ozon entspricht, wobei die erste Bewegungsbahn länger ist als die zweite.

30. Vorrichtung nach einem der vorhergehenden Ansprüche, die des Weiteren einen Schwingungserzeuger umfasst, mit dem ein erster (50) und ein zweiter (55) elektrostatischer Abscheider in Schwingung versetzt und Verschmutzungen und/oder Feststoffpartikel von dem ersten (50) und dem zweiten (55) elektrostatischen Abscheider entfernt werden können.

31. Verfahren für das Reinigen eines Luftstroms, das Folgendes umfasst:
(a) Vorbeiströmenlassen des Luftstroms an einer Ultraviolettlichtquelle (25), die so stark ist, dass in dem Luftstrom Ozon entsteht, und
(b) Regulieren einer Menge an auf den Luftstrom fallendem Ozon durch Ermitteln eines Verschmutzungspegels oder eines Ozonpegels in dem Luftstrom und Regulieren einer Beleuchtung der Ultraviolettlichtquelle (25) als Reaktion auf die Menge an Verschmutzungen und die Menge an Ozon durch Regulieren des Stroms von einer Stromversorgung für die Ultraviolettlichtquelle,
und **dadurch gekennzeichnet, dass** das Regulieren das Modulieren des auf den Luftstrom fallenden Ozons beinhaltet,
wobei das Modulieren durch eine Modulierstruktur (30) erfolgt, die das auf den Luftstrom fallende Ozon moduliert.

32. Verfahren nach Anspruch 31, das des Weiteren das Erfassen des Verschmutzungspegels des Luftstroms umfasst und bei dem das Modulieren dadurch erfolgt, dass die Modulierstruktur (30) als Reaktion auf den Verschmutzungspegel verschoben wird.

33. Verfahren nach Anspruch 31, das des Weiteren das Erfassen des Ozonpegels des Luftstroms umfasst und bei dem das Modulieren dadurch erfolgt, dass die Modulierstruktur (30) als Reaktion auf den Ozonpegel verschoben wird.

34. Verfahren nach Anspruch 31, bei dem das Modulieren durch eine Modulierstruktur (30) bewirkt wird, die aus einer Gruppe ausgewählt wird, die aus einem Leitblech (30), einem Katalysator (100), einer Abschirmung (112), einem Zwischenelement (120) und einer beliebigen Kombination davon besteht.

35. Verfahren nach einem der Ansprüche 31-34, das des Weiteren das Regulieren einer Bewegungsbahn des Luftstroms umfasst.

36. Verfahren nach Anspruch 31, bei dem die Modulierstruktur (30) des Weiteren einen Katalysator (100) umfasst und zu dem des Weiteren das Regulieren eines Abstandes zwischen dem Katalysator (100) und der Ultraviolettlichtquelle (25) gehört.

37. Verfahren nach Anspruch 31, bei dem die Modulierstruktur (30) des Weiteren einen Katalysator (100) umfasst und ein Zwischenelement (120), das zwischen der Ultraviolettlichtquelle (25) und dem Katalysator (100) angeordnet ist, wobei zu dem Verfahren des Weiteren das Verschieben des Zwischenelements (120) gehört, um die von der Ultraviolettlichtquelle (25) emittierte Strahlung, die auf den Katalysator (100) fällt, zu ändern.

38. Verfahren nach einem der Ansprüche 31-37, bei dem Schritt (b) den Strom für die Ultraviolettlichtquelle (25) reguliert.

39. Verfahren nach einem der Ansprüche 31-38, bei dem Schritt (b) eine Geschwindigkeit des Luftstroms reguliert.

40. Verfahren nach einem der Ansprüche 31-39, bei dem es sich bei der Ultraviolettlichtquelle (25) um eine Ultraviolettlampe handelt, die energiereiche Photonen emittiert.

41. Verfahren nach einem der Ansprüche 31-40, bei dem die Ultraviolettlichtquelle (25) Strahlung mit einer geringeren Wellenlänge als ungefähr 200 nm emittiert.

42. Verfahren nach einem der Ansprüche 31-40, bei dem die Wellenlänge in einem Bereich von ungefähr 185 nm bis 254 nm liegt.

43. Verfahren nach Anspruch 31, 32 oder 33, bei dem die Modulierstruktur (30) eine Leitblechanordnung (30) umfasst, die in einem Lüftungskanal (10) angeordnet ist, und zu dem des Weiteren das Steuern der Leitblechanordnung (30) gehört, so dass die Länge einer Bewegungsbahn des Luftstroms geändert wird.

44. Verfahren nach Anspruch 31, bei dem die Modulierstruktur (30) aus einer Gruppe ausgewählt wird, die aus einem Leitblech (30), einem Katalysator (100), einem Zwischenelement (120) und einer beliebigen Kombination davon besteht.

45. Verfahren nach Anspruch 44, bei dem der Katalysator (100) aus einer Gruppe ausgewählt wird, die aus Titandioxid, einem Ultraviolettlicht reflektierenden Material, das die von der Ultraviolettlichtquelle (25) erzeugte Ozonmenge erhöht, und einer beliebigen Kombination davon besteht.

46. Verfahren nach Anspruch 44, bei dem das Leitblech (30) eine Anordnung aufweist und sich das Leitblech (30) so öffnen beziehungsweise schließen lässt, dass sich die Länge einer Bewegungsbahn des auf das Ozon fallenden Luftstroms ändert.

47. Verfahren nach Anspruch 45, bei dem der Katalysator (100) den Einfluss der Ultraviolettlichtquelle (25) ändert, um eine Menge des Ozons zu regulieren, das auf den Luftstrom fällt.

48. Verfahren nach Anspruch 31, bei dem des Weiteren ein Katalysator (100) und/ oder die Ultraviolettlichtquelle (25) in Bezug zueinander verschoben werden, um den Einfluss des Katalysators (100) auf die Ultraviolettlichtquelle (25) zu ändern und das auf den Luftstrom fallende Ozon zu regulieren.

49. Verfahren nach Anspruch 48, bei dem des Weiteren ein Zwischenelement (120) zwischen dem Katalysator (100) und der Ultraviolettlichtquelle (25) platziert wird, wobei sich das Zwischenelement (120) zwischen einer Position für die maximale Ozonerzeugung und einer Position für die minimale Ozonerzeugung verschieben lässt.

50. Verfahren nach einem der Ansprüche 31-49, das des Weiteren das Abschirmen der Ultraviolettlichtquelle (25) von dem Luftstrom umfasst, damit die Temperatur der Ultraviolettlichtquelle (25) zumindest aufrechterhalten wird.

51. Verfahren nach Anspruch 44, das des Weiteren das Betätigen der Modulierstruktur (30) als Reaktion auf Signale umfasst, so dass eine Position für die maximale Ozonerzeugung und eine Position für die minimale Ozonerzeugung entsteht.

52. Verfahren nach Anspruch 51, bei dem die Signale von einem ersten Sensor (60) und einem zweiten Sensor (65) bereitgestellt werden.

## Revendications

1. Appareil comprenant:
une source de lumière ultraviolette (25) qui peut produire de l'ozone frappant un écoulement d'air dans une conduite de ventilation (10) ; et
un régulateur (12) qui régule l'ozone frappant ledit écoulement d'air, ledit régulateur (12) ayant un circuit de commande (90) connecté de manière opérationnelle à ladite source de lumière ultraviolette (25), dans lequel ledit circuit de commande (90) répond à un premier signal correspondant à un niveau de contamination dudit écoulement d'air ou à un deuxième signal correspondant à un niveau d'ozone dans ledit écoulement d'air et régule un éclairement de ladite source de lumière ultraviolette (25), ledit circuit de commande (90) régulant l'ozone frappant ledit écoulement d'air en régulant la puissance de ladite alimentation sur ladite source de lumière ultraviolette, et **caractérisé en ce que** ledit régulateur comprend une structure de modulation (30, 100; 112; 120) qui module l'ozone frappant ledit écoulement d'air, et ledit circuit de commande est également connecté de façon opérationnelle à ladite structure de modulation.

2. Appareil selon la revendication 1, dans lequel ledit circuit de commande (90) répond à une pluralité de signaux correspondant à un niveau de contamination ou à un niveau d'ozone dudit écoulement d'air pour commander ladite structure de modulation (30).

3. Appareil selon la revendication 1, comprenant en outre un actionneur (40) pour actionner ladite structure de modulation (30) pour moduler l'ozone.

4. Appareil selon la revendication 2, dans lequel ledit régulateur (12) comprend en outre un actionneur (40), et dans lequel ledit circuit de commande (90) répond aux signaux correspondant audit niveau d'ozone ou de contamination dudit écoulement d'air pour commander ledit actionneur (40) afin de transmettre un déplacement de modulation de ladite structure de modulation (30).

5. Appareil selon la revendication 3 ou 4, dans lequel ledit régulateur (12) comprend en outre un ou plusieurs capteurs (60, 65) qui fournissent ladite pluralité de signaux.

6. Appareil selon la revendication 3, 4 ou 5, dans lequel lesdits signaux sont fournis par un premier capteur (60) et un deuxième capteur (65) correspondant respectivement audit niveau de contamination et audit niveau d'ozone dans ladite conduite de ventilation (10).

7. Appareil selon l'une quelconque des revendications précédentes, dans lequel ledit circuit de commande (90) régule en outre l'ozone en actionnant ladite structure de modulation (30) à une position maximale correspondant à une quantité maximale d'ozone frappant ledit écoulement d'air et une position minimale correspondant à une quantité minimale d'ozone frappant ledit écoulement d'air.

8. Appareil selon l'une quelconque des revendications précédentes, dans lequel ladite structure de modulation est sélectionnée parmi le groupe se composant : d'une chicane (30), d'un catalyseur (100), d'un écran (112), d'un élément intermédiaire (120), et d'une combinaison quelconque de ceux-ci.

9. Appareil selon l'une quelconque des revendications précédentes, dans lequel ladite structure de modulation (30) comprend une ou plusieurs chicanes (30) disposées selon un agencement permettant de réguler un trajet de circulation dudit écoulement d'air qui se lie à l'ozone.

10. Appareil selon l'une quelconque des revendications précédentes, dans lequel ladite structure de modulation (30) comprend en outre une pluralité de chicanes (30) disposées dans ladite conduite de ventilation (10) en réseau, et dans lequel ladite pluralité de chicanes (30) s'ouvrent et se ferment pour faire varier une distance d'un trajet de circulation dudit écoulement d'air qui se lie à l'ozone.

11. Appareil selon l'une quelconque des revendications précédentes, dans lequel ladite structure de modulation (30) comprend une pluralité de chicanes (30) qui sont configurables en au moins deux trajets de circulation de distances différentes pour réguler l'ozone.

12. Appareil selon l'une quelconque des revendications 1 à 8, dans lequel ladite structure de modulation (30) comprend un catalyseur (100) qui est disposé dans ladite conduite de ventilation (10), et dans lequel ledit régulateur (12) modifie une exposition de ladite source de lumière ultraviolette (25) sur ledit catalyseur (100) afin de réguler une quantité d'ozone.

13. Appareil selon la revendication 12, dans lequel ledit catalyseur (100) est sélectionné parmi le groupe composé : de dioxyde de titane, d'un matériau réfléchissant la source de lumière ultraviolette qui augmente la quantité d'ozone produite par ladite source de lumière ultraviolette (25), et une combinaison quelconque de ceux-ci.

14. Appareil selon la revendication 12 ou 13, dans lequel ledit catalyseur (100) peut se déplacer entre une position produisant un maximum d'ozone et une position produisant un minimum d'ozone qui est plus éloignée de ladite source de lumière ultraviolette (25) que ladite position produisant le maximum d'ozone.

15. Appareil selon la revendication 12, 13 ou 14, dans lequel ledit régulateur '(12) déplace ledit catalyseur (100) et/ou ladite source de lumière ultraviolette (25) l'un par rapport à l'autre afin de modifier ladite exposition de ladite source de lumière ultraviolette (25) vers ledit catalyseur (100) pour réguler l'ozone frappant l'écoulement d'air.

16. Appareil selon l'une quelconque des revendications 1 à 8, dans lequel ladite structure de modulation (30) comprend un élément intermédiaire (120) disposé entre ladite source de lumière ultraviolette (25) et ledit écoulement d'air.

17. Appareil selon la revendication 16, dans lequel ledit élément intermédiaire (120) a au moins une ouverture (150).

18. Appareil selon la revendication 16 ou 17, dans lequel ledit élément intermédiaire (120) a une pluralité d'ouvertures (150) disposées selon une certaine configuration.

19. Appareil selon la revendication 16, 17 ou 18, dans lequel ladite structure de modulation (30) comprend un élément intermédiaire (120) disposé entre ladite source de lumière ultraviolette (25) et ledit écoulement d'air, ledit élément intermédiaire (120) étant mobile entre une position produisant un maximum d'ozone et une position produisant un minimum d'ozone.

20. Appareil selon l'une quelconque des revendications 1 à 8, dans lequel ladite structure de modulation (30) comprend un écran (112) placé entre ladite source de lumière ultraviolette (25) et ledit écoulement d'air, ledit écran maintenant une température de ladite source de lumière ultraviolette (25).

21. Appareil selon la revendication 20, dans lequel ledit écran (112) est placé en amont de ladite source de lumière ultraviolette (25).

22. Appareil selon l'une quelconque des revendications précédentes, dans lequel ledit circuit de commande (90) régule l'ozone frappant ledit écoulement d'air en basculant ladite source de lumière ultraviolette (25) vers un état sélectionné parmi le groupe consisté: d'un état illuminé, d'un état non illuminé, d'un état illuminé réduit relatif audit état illuminé, et d'une combinaison quelconque de ceux-ci.

23. Appareil selon l'une quelconque des revendications précédentes, dans lequel ledit régulateur (12) comprend en outre un ventilateur (35) pour réguler une vitesse dudit écoulement d'air passant sur ladite source de lumière ultraviolette (25).

24. Appareil selon l'une quelconque des revendications précédentes, dans lequel ladite source de lumière ultraviolette (25) est un tube de lumière ultraviolette qui émet des photons d'énergie.

25. Appareil selon l'une quelconque des revendications précédentes, dans lequel ladite source de lumière ultraviolette (25) émet un rayonnement ayant une longueur d'onde dans la plage comprise entre environ 185 nm et 254 nm.

26. Appareil selon l'un quelconque des revendications 1 à 24, dans lequel ladite source de lumière ultraviolette (25) émet un rayonnement ayant une longueur d'onde de moins de 200 nm.

27. Appareil selon la revendication 1, dans lequel ledit régulateur (12) comprend en outre au moins un capteur (60, 65) disposé dans ladite conduite de ventilation (10), un microprocesseur (90) et une structure de modulation (30), dans lequel ladite structure de modulation (30) est l'une sélectionnée parmi le groupe se composant : d'une chicane (30), d'un catalyseur (100), d'un élément intermédiaire (120) et d'une combinaison quelconque de ceux-ci.

28. Appareil selon la revendication 27, dans lequel ledit microprocesseur (90) répond aux signaux fournis par ledit au moins premier capteur (60, 65) pour actionner ladite structure de modulation (30), en réponse auxdits contaminants, entre une position produisant le maximum d'ozone correspondant à une quantité maximale d'ozone frappant ledit écoulement d'air et une position produisant le minimum d'ozone correspondant à une quantité minimale d'ozone frappant ledit écoulement d'air.

29. Appareil selon la revendication 27 ou 28, dans lequel ladite structure de modulation (30) est un chicane (30), et dans lequel ledit microprocesseur (90) commande un actionneur (40) pour commander ladite chicane (30) formant un premier trajet de circulation correspondant à une quantité maximale d'ozone frappant ledit écoulement d'air et formant un deuxième trajet de circulation correspondant à une quantité minimale d'ozone sur ledit écoulement d'air, dans lequel ledit premier trajet de circulation a une distance supérieure audit deuxième trajet de circulation.

30. Appareil selon l'une quelconque des revendications précédentes, comprenant en outre un vibreur pour faire vibrer un premier (50) et un deuxième (55) dispositifs de précipitation électrostatiques pour retirer une contamination et/ou des particules de matière desdits premier (50) et deuxième (55) dispositifs de précipitation électrostatiques.

31. Procédé de purification d'un écoulement d'air, consistant à :
(a) faire passer l'écoulement d'air par une source de lumière ultraviolette (25) suffisante pour créer de l'ozone dans l'écoulement d'air ; et
(b) faire une régulation d'une quantité d'ozone frappant ledit écoulement d'air par détermination d'un niveau de contaminants ou d'un niveau d'ozone dans l'écoulement d'air et faire une régulation d'un éclairement de ladite source de lumière ultraviolette (25) en réponse. à ladite quantité de contaminants et ladite quantité d'ozone par régulation de puissance à partir d'une alimentation sur ladite source de lumière ultraviolette
et **caractérisé par** ladite étape de régulation comprenant la modulation de l'ozone frappant l'écoulement d'air, dans lequel ladite modulation est effectuée par une structure de modulation (30) qui module l'ozone frappant l'écoulement d'air.

32. Procédé selon la revendication 31, comprenant en outre l'étape de détection dudit niveau de contamination de l'écoulement d'air, et ladite modulation est effectuée par déplacement de ladite structure de modulation (30) en réponse audit niveau de contamination.

33. Procédé selon la revendication 31, comprenant en outre l'étape de détection dudit niveau d'ozone de l'écoulement d'air, et ladite modulation est effectuée par déplacement de ladite structure de modulation (30) en réponse audit niveau d'ozone.

34. Procédé selon la revendication 31, dans lequel ladite modulation est effectuée par une structure de modulation (30) sélectionnée parmi le groupe se composant: d'une chicane (30), d'un catalyseur (100), d'un écran (112), d'un élément intermédiaire (120) et d'une combinaison quelconque de ceux-ci.

35. Procédé selon l'une quelconque des revendications 31 à 34, comprenant en outre l'étape de régulation d'un trajet de circulation dudit écoulement d'air.

36. Procédé selon la revendication 31, dans lequel ladite structure de modulation (30) comprend en outre un catalyseur (100), et dans lequel le procédé a en outre l'étape de régulation d'une distance dudit catalyseur (100) et de ladite source de lumière ultraviolette (25) l'un par rapport à l'autre.

37. Procédé selon la revendication 31, dans lequel ladite structure de modulation (30) comprend en outre un catalyseur (100) et un élément intermédiaire (120), disposé entre ladite source de lumière ultraviolette (25) et ledit catalyseur (100), et dans lequel le procédé comprend en outre l'étape de déplacement dudit élément intermédiaire (120) afin de faire varier le rayonnement émis par ladite source de lumière ultraviolette (25) qui frappe ledit catalyseur (100).

38. Procédé selon l'une quelconque, des revendications 31 à 37, dans lequel l'étape (b) régule la puissance à ladite source de lumière ultraviolette (25).

39. Procédé selon l'une quelconque des revendications 31 à 38, dans lequel l'étape (b) régule une vitesse de l'écoulement d'air.

40. Procédé selon l'une quelconque des revendications 31 à 39, dans lequel ladite source de lumière ultraviolette (25) est un tube de lumière ultraviolette qui émet des photons d'énergie.

41. Procédé selon l'une quelconque des revendications 31 à 40, dans lequel ladite source de lumière ultraviolette (25) émet un rayonnement ayant une longueur d'onde qui est inférieure à environ 200 nm.

42. Procédé selon l'une quelconque des revendications 31 à 40, dans lequel ladite longueur d'onde est dans une plage comprise entre une longueur d'onde de 185nm et une longueur d'onde de 254 nm .

43. Procédé selon la revendication 31, 32 ou 33, dans lequel ladite structure de modulation (30) comprend une disposition de chicane (30) placée dans une conduite de ventilation (10), et dans lequel le procédé a en outre l'étape de commande de ladite disposition de chicane (30) pour faire varier la distance d'un trajet de circulation de l'écoulement d'air.

44. Procédé selon la revendication 31, dans lequel ladite structure de modulation (30) est sélectionnée parmi le groupe se composant : d'une chicane (30), d'un catalyseur (100), d'un élément intermédiaire (120), et d'une combinaison quelconque de ceux-ci.

45. Procédé selon la revendication 44, dans lequel ledit catalyseur (100) est sélectionné parmi le groupe se composant : d'oxyde de titane, d'un matériau réfléchissant la lumière ultraviolette qui augmente la quantité d'ozone produite par ladite source de lumière ultraviolette (25) et de toutes combinaisons de ceux-ci.

46. Procédé selon la revendication 44, dans lequel ladite chicane (30) est disposée en réseau, et dans lequel ladite chicane (30) s'ouvre et se ferme pour modifier la distance d'un trajet de circulation de l'écoulement d'air qui est frappé par l'ozone.

47. Procédé selon la revendication 45, dans lequel ledit catalyseur (100) modifie une exposition de ladite source de lumière ultraviolette (25) afin de réguler une quantité d'ozone frappant ledit écoulement d'air.

48. Procédé selon la revendication 31, comprenant en outre l'étape de déplacement d'un catalyseur (100) et/ou de ladite source de lumière ultraviolette (25) l'un par rapport à l'autre pour modifier une exposition de ladite source de lumière ultraviolette (25) sur ledit catalyseur (100) afin de réguler l'ozone frappant l'écoulement d'air.

49. Procédé selon la revendication 48, comprenant en outre l'étape consistant à placer un élément intermédiaire (120) entre ledit catalyseur (100) et ladite source de lumière ultraviolette, (25), ledit élément intermédiaire (120) étant mobile entre une position produisant un maximum d'ozone et une position produisant un minimum d'ozone.

50. Procédé selon l'une quelconque des revendications 31 à 49, comprenant en outre l'étape consistant à protéger ladite source de lumière ultraviolette (25) dudit écoulement d'air pour au moins maintenir une température de ladite source de lumière ultraviolette (25).

51. Procédé selon la revendication 44, comprenant en outre l'étape consistant à actionner ladite structure de modulation (30) en réponse à des signaux pour former une position de production maximum d'ozone, et pour former une position de production minimum d'ozone.

52. Procédé selon la revendication 51, dans lequel lesdits signaux sont fournis par un premier capteur (60) et un deuxième capteur (65).
